## Europäisches Patentamt
## European Patent Office
## Office européen des brevets

(19)

(11) Publication number: **0 225 658**
**B1**

(12)

# EUROPEAN PATENT SPECIFICATION

(45) Date of publication of the patent specification:
**28.02.90**

(51) Int. Cl.⁴: **A61K 7/30,** C11D 17/00, C11D 3/20

(21) Application number: **86201947.8**

(22) Date of filing: **07.11.86**

(54) **Shaped cleansing composition.**

(30) Priority: **12.11.85 NL 8503100**

(43) Date of publication of application:
**16.06.87 Bulletin 87/25**

(45) Publication of the grant of the patent:
**28.02.90 Bulletin 90/9**

(84) Designated Contracting States:
**AT BE CH DE ES FR GB GR IT LI LU NL SE**

(56) References cited:
**CH-A- 352 462
DE-B- 1 012 034
GB-A- 1 052 796
GB-A- 2 095 694**

(73) Proprietor: **Douwe Egberts Koninklijke Tabaksfabriek-Koffiebranderijen-Theehandel N.V., Leeuwarderweg 1, NL-8501 ZD Joure(NL)**

(72) Inventor: **Zaagman, Daniel, Johan Vermeerstraat 20, NL-2681 NV Monster(NL)**

(74) Representative: **Smulders, Theodorus A.H.J., Ir. et al, Vereenigde Octrooibureaux Nieuwe Parklaan 107, NL-2587 BP 's-Gravenhage(NL)**

ACTORUM AG

## Description

The invention relates to a shaped cleansing composition containing an alkali, alkaline earth or ammonium perborate anhydride and at least one cleansing constituent.

A composition of the type indicated above is disclosed in NL-B 101 468. It describes a process of preparing a tabletted denture cleanser by compressing a water soluble cleansing constituent and a perborate anhydride into the form of a tablet. Tablets thus obtained rapidly disassociate in water due to evolution of gas to form a cleansing solution.

A disadvantage to perborate anhydride is that it is hygroscopic, so that during production and storage of the tablets exposure to air should be avoided. The tablets can therefore only be preserved in properly sealed packaging material. In use, however, when exposed to air, the tablets tend to go soft, stick together and only slowly dissolve.

These drawbacks are not shown by the present cleansing composition. The invention consists in that the above well-known shaped cleansing composition contains an alkyl salicylate of which the alkyl group has 1 to 4 carbon atoms.

It is preferred that use should be made of methyl salicylate. Methyl salicylate is a well-known fragrance providing agent which also acts as a disinfectant and goes into a clear solution without leaving a film.

The proportion of alkyl salicylate in the cleansing composition may be varied over a wide range. To prevent the tablets from going soft it is generally desirable that the alkyl salicylate should be incorporated in the cleansing composition in an amount of 0,25% by weight. Alkyl salicylate also effects an increase in the rate of solubility of the tablet in water. Upon addition of unduly large amounts of alkyl salicylate, however, the rate of solubility will decrease. In view of these effects the cleansing composition contains alkyl salicylate preferably in an amount of 0,25 to 5, more particularly 0,5 to 1,5 per cent by weight, based on the total cleansing composition.

The perborate anhydride, also referred to as oxoborate, is a special form of perborate which upon contact with water splits off gaseous oxygen in an amount of at least 15%. It is formed upon thermal dehydration of perborate monohydrate (see Ullmann Encyklopädie der Technischen Chemie, Volume 17, page 719 and DE-C 528 873).

The bleaching agent often used in the detergents industry, which is generally referred to as perborate, differs from the perborate anhydride in that it formally contains four molecules of crystal water and splits off reactive hydrogen peroxide in water. Upon dissolution in water there will be no appreciable formation of gas. Nor will such be the case upon dissolution of perborate monohydrate, which just as the perborate tetrahydrate is a bleaching agent and is formed as a result of partial dehydration of the tetrahydrate.

It should be added that US-A 3 337 466 (Table in Column 4) mentions a composition containing sodium perborate and methyl salicylate. What is meant there is sodium perborate tetrahydrate and not sodium perborate anhydride, which is clear from column 7, lines 50-53, where it says that the sodium perborate contains 10% of available oxygen. Sodium perborate tetrahydrate is known to contain such an amount of oxygen (see Encyclopedia of Industrial Chemical Analysis, Vol. 7, p. 379, Table 20; 1968 Edition). In said composition (see footnote with the table) methyl salicylate is present as a fragrance providing agent. Similar compositions are disclosed in GB-A 1 052 796 according to which methyl salicylate is also added as a fragrance.

The amount of alkali, alkaline earth or ammonium perborate anhydride may be varied over a wide range. The composition generally contains 5 to 50 per cent by weight of perborate anhydride. It is preferred that it should contain 15 to 25 per cent by weight, more particularly 17 to 19 per cent by weight of perborate anhydride. Use of a too low percentage will cause the tablet to disassociate only slowly. Use of a too high percentage will be attended with such a rapid evolution of oxygen gas that the tablet will be caused to float. The gas will then directly escape into the air, so that the cleansing constituents cannot be properly stirred and promptly dissolved in water.

The size of the perborate anhydride particles to be used in the preparation of the present cleansing composition is not critical. However, the disassociation of the cleansing tablets is promoted by choosing particles that are sufficiently small. It is preferred that the particles should have an average diameter in the range of 200 to 500 µm. As examples of suitable alkali and earth alkali perborate anhydrides may be mentioned sodium perborate anhydride, lithium perborate anhydride, potassium perborate anhydride and calcium perborate anhydride.

It is preferred that use should be made of sodium perborate anhydride. The present cleansing composition contains at least one cleansing constituent. The choice of the cleansing constituents will depend on the cleansing effect envisaged. An important use of the present cleansing composition is the cleansing of dentures, artificial teeth, orthodontic bridges, denture plates, etc. They need regular cleansing in order to avoid discoloration and staining by foods, tobacco, etc. and the growth of bacteria and plaque on the denture surface. Moreover, in the long run tartar will be formed on the denture.

It is often practice for dentures to be cleaned with a tooth brush and an abrasive-containing toothpaste. This may lead to increased wear on dentures. Such wear will hardly be caused by chemical cleaning. In that case the dentures are regularly (for instance for 15 to 30 minutes daily) immersed in a cleanser solution. The cleanser solution is usually prepared by dissolving a cleansing tablet (2 to 10 g) in water (50 to 300 ml).

As examples of suitable cleansing constituents that may be incorporated in the cleansing composition may be mentioned bleaches, sequestering agents, disinfectants, enzymes and surface active compounds. Optionally, the cleansing composition may also contain fillers, tabletting aids such as release agents, lubricants, anti-sticking agents and anti-caking agents.

As described hereinafter, these agents have different functions and are therefore generally applied in combination. Bleaching agents are added for the purpose of removing stains and discoloration caused by fruit, tea, coffee, tobacco smoke, etc. As examples of suitable bleaches may be mentioned sodium perborate, monohydrate, sodium perborate tetrahydrate, magnesium monoperoxyphthalate, sodium dichlorisocyanurate, sodium hypochlorite and salts of perbenzoic acid.

The bleaching agent will generally be used in an amount of 1 to 10 per cent by weight, calculated on the total composition. A very suitable bleaching agent for dentures is sodium perborate monohydrate, which unlike for instance sodium hypochlorite will not exert harmful effects on metals employed in denture constructions.

The present composition generally also contains one or more sequestering agents, particularly for dissolving calcium-containing deposits. Well-known sequestering agents are sodium tripolyphosphate, sodium ethylene diamine tetra-acetate, sodium nitrolo-tri-acetate, alkyl phosphonates, polycarboxylates and polyhydroxy carboxylates and polycarboxylic acids such as citric acid. The sequestering agents are generally used in an amount of 5 to 50 per cent by weight, based on the total composition. Sodium tripolyphosphate is a very suitable sequestering agent because it constitutes an alkaline buffer for the cleansing solution and consequently promotes the action of peracid bleaches.

Cleansing compositions for dentures generally also contain a disinfectant. Alkyl salicylate as such is a very suitable agent. It reduces (microbial) plaque on dentures, improves the condition of a mucous membrane inflamed as a result of wearing a denture. Optionally, the cleansing composition may contain other disinfectants, such as hexachlorophene. Chlorhexidine gluconate is a less suitable cleansing agent in that unlike methyl salicylate it may cause stains on the denture surface.

Bacterial plaque on the teeth also may be inhibited by the use in the composition of enzymes, which will break down proteins and polysaccharides of plaque. A suitable enzyme-containing preparation is known under the name of Alcalase 243®, which may be present in an amount of 1 to 20 per cent by weight, based on the total composition.

The present composition also may contain a small proportion (generally 0,1 to 10 per cent by weight, based on the total cleansing composition) of surface active compounds. Such compounds promote the formation of froth in the cleansing solution and the contact of this solution with the surface to be cleansed. Examples of suitable anionic surface active compounds include sodium lauryl sulphate and sodium lauryl ether sulphate .

As suitable nonionic surface active compounds may be mentioned tallow fatty alcohol ethoxylates and alkyl phenol ethoxylates. As suitable cationic surface active compounds may be mentioned dimethyl dialkyl ammonium chloride and dimethyl dialkyl benzoyl ammonium chloride.

Cationic compounds, however, are less suitable to be used in combination with enzymes in that they give rise then to precipitation.

The present cleansing composition is also particularly suitable for cleansing surfaces that are difficult to clean with a brush, such as the inside of a coffee pot, a thermosflask and a decanter.

Optionally, the cleansing composition may contain one or more fillers, such as sodium chloride, sodium sulphate, potassium chloride, potassium sulphate, saccharose, sodium carbonate and dicalcium phosphate.

In addition to cleansing agents and fillers the cleansing composition generally also contains binding agents, release agents, lubricating agents and anti-caking agents. These agents facilitate the shaping of the cleansing composition.

As suitable binders may be mentioned polyethylene glycol, gelatin, polyvinyl pyrrolidone, arabic gum and various cellulose derivatives. Examples of suitable release agents include magnesium stearate and talcum. Examples of suitable lubricants include petrolatum and sodium lauryl sulphate. A suitable anti-caking agent is aerosil.

After the various components of the composition have been mixed, they may be compressed into the form of a tablet.

Alternatively, the cleansing composition may be shaped into the form of a pill, a pellet or a granulate. The invention will be further described in the following examples.

## Example 1

Cleansing tablets of the following composition were prepared as described below:

| | |
|---|---|
| sodium tripolyphosphate | 35.0 g |
| sodium lauryl sulphate | 0.25 g |
| methyl salicylate resp. 0, 0.25, 0.5, 0.75, 1.0, 1.25 | 1.5 g |
| sodium perborate anhydride | 18.5 g |
| polyethylene glycol (average mol. wt 12000) | 4.0 g |
| microbial protease | 1 g |
| sodium chloride | balance up to 100 g |

The polyethylene glycol was dissolved in two parts of hot acetone, to which also the methyl salicylate and sodium lauryl sulphate were added. After this solution had been mixed with the perborate anhydride, the acetone was evaporated off and the resulting powder was mixed (after sieving, about 1 mm mesh) with the other components. The free-flowing powder mixture thus obtained could without difficulty be compressed into 3 g tablets 18 mm in diameter.

## Example 2

Cleansing tablets of the following composition were prepared in accordance with the procedure described in Example 1.

| | |
|---|---|
| Sodium tripolyphosphate | 30.0 g |
| sodium lauryl sulphate | 0.2 g |
| ethyl salicylate | 1.25 g |
| sodium perborate anhydride | 18.0 g |
| polyethylene glycol (average mol. wt 10000) | 3.0 g |
| microbial protease | 0.4 g |
| sodium chloride | balance up to 100 g |

## Example 3

Tablets of Example 1 containing different amounts of methyl salicylate were kept in an exsiccator at room temperature and at constant relative humidity. Every thirty minutes the tablets were checked for stickiness.
The results are given in the table below.

| methyl salicylate in wt.% | 0 | 0.25 | 0.5 | 0.75 | 1,0 | 1.25 | 1.5 |
|---|---|---|---|---|---|---|---|
| sticking time in hours at a rel. humidity of 56% | 11/2 | 21/2 | 21/2 | 5 | 5 | 7 | 7 |
| sticking time in hours at a rel. humidity of 81% | 1/2 | 1/2 | 1/2 | 11/2 | 2 | 31/2 | 31/2 |

A tablet which contains for instance 1 per cent by weight of methyl salicylate is still usable when it has been in a damp bath room for a long time after removal of the packaging material.

### Example 4

Tablets of Example 1, which contain different amounts of methyl salicylate, were dissolved in 150 ml of water of 50°C. The dissolving times are given in the table below.

| methyl salicylate | 0 | 0.25 | 0.5 | 1.0 | 1.25 | 1.5 |
|---|---|---|---|---|---|---|
| dissolving time in sec. | 35 | 35 | 30 | 25 | 45 | 60 |

The results show that the dissolving time first decreases and subsequently increases with increasing amount of methyl salicylate.

### Claims

1. A shaped cleansing composition containing an alkali, alkaline earth or ammonium perborate anhydride and at least one cleansing constituent, characterized in that the cleansing composition contains an alkyl salicylate of which the alkyl group has 1 to 4 carbon atoms.

2. A shaped cleansing composition according to claim 1, characterized in that the alkyl group is a methyl group.

3. A shaped cleansing composition according to claim 1 or 2, characterized in that the salicylate is present in an amount of 0,25 to 5 per cent by weight, based on the total composition.

4. A shaped cleansing composition according to claim 3, characterized in that the salicylate is present in an amount of 0,5 to 1,5 per cent by weight, based on the total composition.

### Patentansprüche

1. Geformtes Reinigungsmittel, enthaltend ein Alkali-, Erdalkali- oder Ammonium-perborat-anhydrid und mindestens einen Reinigungsbestandteil, dadurch gekennzeichnet, daß das Reinigungsmittel ein Alkylsalicylat enthält, dessen Alkylgruppe 1 bis 4 Kohlenstoffatome hat.

2. Geformtes Reinigungsmittel nach Anspruch 1, dadurch gekennzeichnet, daß die Alkylgruppe eine Methylgruppe ist.

3. Geformtes Reinigungsmittel nach Anspruch 1 oder 2, dadurch gekennzeichnet, daß das Salicylat in einer Menge von 0,25 bis 5 Gew.-%, basierend auf der Gesamt-Zusammensetzung, vorhanden ist.

4. Geformtes Reinigungsmittel nach Anspruch 3, dadurch gekennzeichnet, daß das Salicylat in einer Menge von 0,5 bis 1,5 Gew.-%, basierend auf der Gesamt-Zusammensetzung, vorhanden ist.

### Revendications

1. Un produit (composition) de nettoyage façonné contenant un anhydride de perborate d'un métal alcalin ou alcalino-terreux ou d'ammonium avec un ou plusieurs agents de nettoyage, produit caractérisé en ce qu'il comprend un salicylate d'alkyle dont l'alkyle a de 1 à 4 atomes de carbone.

2. Une composition de nettoyage façonnée selon la revendication 1, caractérisée en ce que le salicylate d'alkyle est le salicylate de méthyle.

3. Une composition de nettoyage selon la revendication 1 ou 2, caractérisée en ce que la proportion du salicylate est de 0,25 à 5% du poids total de la composition.

4. Une composition de nettoyage selon la revendication 3, caractérisée en ce que la proportion du salicylate est de 0,5 à 1,5% du poids total de la composition.